# EUROPEAN PATENT APPLICATION

(11) **EP 2 347 763 A1**
(43) Date of publication of application: **27.07.2011**
(21) Application number: 09816502.0
(22) Date of filing: 13.02.2009
(51) Int. Cl.: A61K 38/39, A61K 35/12, A61K 35/14, A61P 25/00

(54) **AN IMPLANTABLE NEUROENDOPROSTHESIS SYSTEM, A METHOD FOR PREPARING SAME AND A PROCEDURE FOR PERFORMING OF A RECONSTRUCTIVE NEUROSURGICAL OPERATION**

(30) Priority: 25.09.2008 RU 2008138161
(71) Applicant: Bryukhovetskiy, Andrey Stepanovich, Moscow 117437 (RU)
(72) Inventor: SEVASTIANOV, Viktor Ivanovich, Moscow 125480 (RU)
(74) Representative: Boeckh, Tobias
(86) International application number: PCT/RU2009/000067
(87) International publication number: WO 2010/036141

(57) **Abstract**

The invention is used in neurosurgery and tissue engineering of organs to replace defects of nervous tissue of a mammal brain and spinal cord in reconstructive neurosurgical operations. The object of invention is to develop a tissue-replaceable artificial cell-biopolymer neuroendoprosthetic system (ACBP NEPS) for surgical plasty of defects of nervous tissue of brain and spinal cord that will stimulate regeneration and growth of damaged axons of neural cells, as well as to develop a production method of the ACBP NEPS and a method of reconstructive neurosurgical operation using the ACBP NEPS. The ACBP NEPS is an elastic cell-biopolymer biologically active mass produced from a heterogeneous collagen-containing matrix for implantation and a biocomposition of cell preparations of various types of autologous cells of a patient. The biocomposition comprises, in a NaCl solution, neural stem cells (NSC), neuroglial ensheathing cells (NGEC), endothelial cells with CD34+ marker (EC) and purified mononuclears (MN) in the following ratios (in parts according to numbers of the cells): 0.8 to 1.2 of NSC; 1.6 to 2.4 of NGEC; 4 to 6 of EC; 4000 to 6000 of MN. The method of production of the ACBP NEPS comprises perfusion of the biocomposition of the cell preparations of various types of autologous cells of a patient into the heterogeneous collagen-containing matrix for implantation to obtain the elastic cell-biopolymer biologically active mass. The method of the reconstructive neurosurgical operation to replace the defects of a mammal brain and/or spinal cord and/or vegetative nervous system comprises implantation of the ACBP NEPS into the defect of neural tissue.

## Description

### Technical Field

The invention relates to the field of neurosurgery and tissue engineering of organs and can be applied to replace defects of neural tissue of brain and spinal cord in reconstructive and repairing therapy of consequences of traumatic, ischemic injuries, surgical operations of central nervous system (CNS) and vegetative nervous system (VNS) of a mammal, for example a human.

### Background Art

Till the end of 20^{th} century, prosthesis of nervous tissue defects was considered impossible and almost unsolvable that can be explained by dogmatic understanding of limited restoration capacities of nervous tissue by brain researchers, neurologists and neurosurgeons as well as by dogmatic notion established by Santiago Ramon-y-Cajal that neural cells were inherently unable to regenerate after the injury. However, the last decade of 20^{th} century has considerably changed the approach by accumulation of new scientific evidence of regenerative potential of CNS, reparative properties of neural stem cells (NSC) and conclusive proofs of restoration opportunities of the axons of injured neurons.

It is known in the art a preparation of hematopoietic stem cells (HSC), which is autologous HSC obtained from a patient peripheral blood enriched with cells containing CD34 antigen in the final concentration of (40 to 100)·10⁶ cells/ml. A therapeutic treatment of a brain and spinal cord (SC) traumatic disease is performed by intrathecal or intraventricular administration of the cell preparation to a patient (RU 2283119 C1, A61K35/14, 2006). However, the preparation consisting of solely HSC appeared insufficiently effective in the therapy of brain and SC nervous tissue defects.

It is known a biopolymer prosthesis «NeuroGel»^{™} to fill defects of nervous tissue (S.Woerly, V.D.Doan, F.Evans-Martin, C.G.Paramore, J.D.Peduzzi, "Spinal cord reconstruction using NeuroGel™ implants and functional recovery after chronic injury", J. of Neurosciensce Res. 2001. Vol.66, P.1187-1197). Research proved a possibility of injured axons to grow through biopolymer composition in a mammal SC and restoration of lost brain functions, while stem cells provide favorable conditions for axon regeneration. However, application of the «NeuroGel»^{™} prosthesis proved inefficient in the treatment of brain and SC defects. Moreover, the «NeuroGel»^{™} composition contains agents, prohibited to be used in humans.

It is also known in the art, a multipurpose heterogeneous collagen matrix for implantation, which is an elastic mass prepared of two collagen sources, one being the tissue of vertebrate animal of one class, and the second being the tissue of other class of animals. The matrix consists of two phases: solid represented by microspheres of a mammal tissue collagen and liquid represented by a denatured bird tissue collagen. This heterogeneous collagen-containing matrix was proposed for restoration of injuries of soft tissues and organs by means of implantation (RU 2249462 C1, A61K38/39, 2005). The matrix is accepted as the prototype of the claimed implantable neuroendoprosthetic system. Implantation of this collagen matrix into a defect of nervous tissue has not activated regenerative potential of the artificial implant in full due to big size of micorspheres (300 to 400µm) and their hardness that led to mechanical damage and death of transplanted stem cells.

### Summary of Invention

An object of the present invention is to provide a tissue replaceable artificial cell-biopolymer neuroendoprosthetic system (ACBP NEPS) for surgical plasty of nervous tissue defects of brain and SC, that will stimulate regeneration and growth of axons of neural cells, as well as to provide a production method of this system and a method of reconstructive neurosurgical operation to repair the defects of brain and/or SC and/or a VNS in a mammal with this system hereinafter referred as the implantable neuroendoprosthetic system or ACBP NEPS.

Said object is reached by that, according to the present invention, it is provided an implantable neuroendoprosthetic system to replace defects of a brain, spinal cord and vegetative nervous system in a mammal in reconstructive neurosurgical operations, wherein the system is an elastic cell-biopolymer biologically active mass produced from a heterogeneous collagen-containing matrix for implantation and a biocomposition of cell preparations of various types of autologous cells of a patient.

More specifically, said biocomposition comprises, in a NaCl solution, neural stem cells (NSC), neuroglial ensheathing cells (NGEC), endothelial cells with CD34+ marker (EC) and purified mononuclears (MN) in the following ratios (in parts according to numbers of the cells): 0.8 to 1.2 of NSC; 1.6 to 2.4 of NGEC; 4 to 6 of EC; 4000 to 6000 of MN. Preferasbly, 0.5 to 1.3% solution of NaCl is used. As the heterogeneous collagen matrix, a composition of a heterogeneous implantable gel *Sphero*®GEL is used.

Said biocomposition can further comprise stimulators of cell regeneration, nerve growth factors and vascular growth factors. The most appropriate, efficient and safe (in consideration of swelling ratio of the *Sphero*®GEL matrix) is the ACBP NEPS in which numbers of said cells contained in 0.5 to 1 ml of 0.9% NaCl solution per 1 ml of said heterogeneous collagen-containing matrix are as follows: 10⁶ of NSC; 2·10⁶ of NGEC; 5·10⁶ of EC; 5·10⁹ of MN and which comprises 0.1 to 0.2 ml of standard solution of a cell regeneration stimulator (methyluracil, leucogen, ATP, pentoxyl etc.).

Said object is also reached by that it is provided a method of production of an implantable neuroendoprosthetic system to replace defects of a brain, spinal cord and vegetative nervous system in a mammal in reconstructive neurosurgical operations, comprising perfusion of a biocomposition of cell preparations of various types of autologous cells of a patient into a heterogeneous collagen-containing matrix for implantation to obtain an elastic cell-biopolymer biologically active mass.

Preferably, the perfusion is performed by centrifugation. The centrifugation is carried out within 1.5 to 2.5 minutes at 1,500 to 25,000 revolutions per minute. Said biocomposition is prepared from cryopreserved cell preparations that, immediately before production of said implantable neuroendoprosthetic system, are thawed by a water bath at 37 to 40°C and then washed at least twice in a physiological NaCl solution.

More specifically, said biocomposition comprises, in a NaCl solution, neural stem cells (NSC), neuroglial ensheathing cells (NGEC), endothelial cells with CD34+ marker (EC) and purified mononuclears (MN). A source of the NSC and the NGEC is preferably olfactory sheath of a nose of a patient, and a source of the EC and the MN is either a bone marrow of the patient or a leukoconcentrate of mobilized autologous stem cells of the patient, wherein the leukoconcentrate is obtained during separation of the patient's peripheral blood after patient stimulation with a granulocyte colony-stimulating factor. Stimulators of tissue regeneration, nerve growth factors and vascular growth factors can be added in said biocomposition. The method of the ACBP NEPS production of the present invention is carried out in sterile conditions either directly in an operation room (ex tempore), or in a culture laboratory (period of implantation up to 6 hours).

Said object is also reached by that it is provided a method of a reconstructive neurosurgical operation to replace defects of a brain, spinal cord and/or vegetative nervous system in a mammal, comprising implantation of a neuroprosthetic system in the form of an elastic cell-biopolymer biologically active mass into a defect of neural tissue, wherein said mass is obtained from a heterogeneous collagen-containing matrix for implantation and a biocomposition of various cell preparations of various types of autologous cells of a patient.

In more detail, said biocomposition said biocomposition comprises, in a NaCl solution, neural stem cells (NSC), neuroglial ensheathing cells (NGEC), endothelial cells with CD34+ marker (EC) and purified mononuclears (MN). A composition of a heterogeneous implantable gel *Sphero*®GEL is preferably used as said heterogeneous collagen-containing matrix. The neuroendoprosthetic system is implanted by its placing into the defect and by filling a whole volume of a cyst or a lesion of the brain and/or spinal cord with the system. After the neuroendoprosthetic system has been placed into the defect, the system is covered with an autologous muscle fascia or an artificial dura mater and/or a biodegradable synthetic polymer coat to reduce a contact of the neuroendoprosthetic system with cerebrospinal fluid of the patient. An implantable biopolymer membrane ElastoPOB® is preferably used as said biodegradable synthetic polymer coat. In case of a complete anatomical dissection of the spinal cord, the neurondoprosthetic system is implanted in the following way: a conduit is formed from an artifical arterial graft and the neurondoprosthetic system by which, at least partially, said graft is filled, the conduit is then placed in a gap between ends of the injured spinal cord and then pia mater of distal and proximal ends of the injured spinal cord is sutured to the conduit walls. The length of the artificial arterial graft is equal to the length of said gap, and the width of the graft is equal to the diameter of the spinal cord in the lesion site. The neuroendoprosthetic system is isolated from a direct aggressive influence of cerebrospinal fluid in an intramedullar or intracerebral implantation.

The implantable biopolymer membrane ElastoPOB® is produced by ZAO Biomir-Servis (Moscow) according to technical standards TY 9398-002-54969743-2006, registration certificate No. φC 01032006/5581-06 of Dec. 28, 2006.

### Brief Description of Drawings

Efficiency of the ACBP NEPS of the present invention is illustrated by the following figures:
Fig. 1 shows a diagram demonstrating efficiency of the treatment of patients with a traumatic brain and spinal cord injury by the method of the present invention and conventional method;
Fig. 2 shows urograms of a patient before (A) and after (B) implantation of the ACBP NEPS according to the present invention.

### Description of Embodiments

Body and structure of the proposed ACBP NEPS provide adequate modeling of a neuroimplant in damaged regions of brain and spinal cord and local stimulation of regeneration processes in the brain and spinal cord lesion sites. Application of only autologous cells preparations in the ACBP NEPS averts problems of histocompatibility and immune conflict, as well as need of using immunosuppressants. Ethic, legal and moral limitations are absent as the therapy involves only autologous cells of the patient. A risk of infectious and viral contamination is excluded, as well as a risk of prion infection possible in case of application of xenogenous materials. Exact number of the applied ACBP NEPS is defined depending on volume, character and location of a brain/spinal cord lesion.

Basic element of the proposed ACBP NEPS development was invention and production of a polymer matrix that would meet specific demands of "bridging" the gap in the damaged regions of brain/spinal cord, provide support and nutrition for the cell preparations, function as orienting vector for the axons of the injured autoneurons and neuroglial cells, stimulate regeneration of the injured axons, contribute to their growth through the defect region of nervous tissue. The proposed matrix should meet safety criteria and biodegrade into safe components within a preset time period (8 to 12 months) being replaced by restored nervous tissue.

The preferable matrix to be used in the proposed ACBP NEPS is the composition of the heterogeneous implantable gel trading as *Sphero*®GEL (Russian trademark registration No. 269774) and produced by ZAO Biomir-Servis (Moscow) according to technical standards TY 9398-001-54969743-2006 of Dec. 26, 2006, registration certificate No. φC 01032006/5580-06 of Dec. 28, 2006.

The *Sphero*®GEL matrix consists of microparticles of cross-linked collagen of type VII obtained from farm animals, which microparticles are suspended in elastic homogeneous gel. The matrix is a unique complex of peptides (30 to 50mg/g), uronic acids (0.8 to 1.2 mg/g) and hexosamines (2.0 to 3.0 mg/g). Amino-acid composition of the matrix is identical to the collagen but hexosamine content exceeds it twice, and uronic acids content is 15 times more. The *Sphero*®GEL matrix is stable for long-term storage and not susceptible to syneresis (expulsion of liquid). In this condition it does not interfuse with water and hydrophobic liquids. At 37°C, the gel turns into liquid interfusing with water and the microparticles of the cross-linked collagen in all ratios. The size of the gel microparticles can vary from 30 to 300 µm. Swelling capacity of the *Sphero*®GEL matrix is no less than 87%, pH = 4.8 to 7.2. Average time of bio resorption in a body varies from several weeks to 9 months depending on the site of implantation and size of the microparticles. It is experimentally and clinically confirmed high biological compatibility and stimulating properties contributing to repairing processes in the site of tissue lesion. Preclinical and clinical trials were performed by the FGU Scientific Research Institute of Transplantology and Artificial Organs, ZAO NeuroVita Clinic, Hospital of the Russian Academy of Sciences (Troitsk), State Clinical Hospital of Civil Aviation, S.P. Botkin's State Clinical Hospital.

The clinical trials demonstrated efficient functional properties of the *Sphero*®GEL matrix applied as:
- Biologically active artificial synovial fluid for therapeutical treatment of knee joints arthroses;
- implants for surgical intervention of peripheral nerves conductance;
- implantable carriers for transplantation and holding stem cells for spinal cord injury therapy.

Just the *Sphero*®GEL matrix was tested on animal models of spinal cord injury in ZAO NeuroVita Clinic for three years and provided the basis to the proposed neuroendoprosthetic system. To establish the cell composition with the stimulators of tissue regeneration, further perfusion of specific cell composition into the *Sphero*®GEL matrix was proposed for experimental and clinical trial of the matrix. The main challenge of the ACBP NEPS development was to select basic components of the cell biocomposition and to determine their concentration in 1 ml of the biodegradable *Sphero*®GEL matrix.

Endothelial cells with CD34+ marker and neural stem cells possess the property of target oriented migration to lesion sites. Transplanted cells form clusters of progenitor cells in brain/spinal cord tissue. This phenomenon became essential in our solution of injured brain/spinal cord regeneration. However, only after long-term animal experiments and 32 surgeries in humans, it has been found the optimal combination of the biopolymer with the cell composition and the regeneration stimulators, that has allowed to achieve maximal clinical and neurophysiological effect of surgical operations in tissue engineering of brain/spinal cord and resulted in the ACBP NEPS development.

The ACBP NEPS is an artificial analogue of nervous tissue and contains all its basic cell elements (neurons, neuroglial cells, endotheliocytes etc.) and the polymer matrix with specified parameters of biodegradation (from 8 to 12 months). Body and plasticity of the ACBP NEPS allow for adequate modeling of neuroimplant in damaged anatomical structures of the brain and also provide local stimulation of regeneration processes in the lesion site. The ACBP NEPS is able to fulfill bridging and nourishing functions to facilitate growth of injured axons through pathologically modified regions of brain/spinal cord tissue.

NSC contained in the biocomposition of the proposed ACBP NEPS can reconstruct injured nervous tissue and first of all provide restoration of gray matter of spinal cord, while NGEC can stimulate differentiation of blood precursor cells into astrocytes and oligodendrocytes that can restore white matter and be a source of the cells remyelinizing damaged axons. Adding hematopoietic stem cells (CD34+CD45-) oriented for endotheliocytic differentiation, hence, presenting the source of EC, to biocomposition enhances development of new vessels, leads to rapid formation of microcapillary vascular network in the endoprosthetis, improves local blood supply. Application, as a component of the cell biocomposition, of stem non-hematopoietic stem cells of mononuclear fraction (MN) of peripheral blood after stimulation by colony-stimulating factors leads to production of a large amount of growth factors that significantly contribute to axon regeneration.

Practical application of various cell preparations in *Sphero*®GEL matrix in clinical practice has showed that best clinical and neurophysiological outcomes are received not from isolated application of the matrix and intracerebral or intramedullar administration of certain cell cultures, but from specific ratio of these cell preparations in *Sphero*®GEL matrix, thus resulting in specific artificial microenvironment of damaged axons, close to natural tissue of developing brain/spinal cord by its cell structure.

Production of cultured NGEC and NSC suspensions follows standard method. The source of these cells is a fragment of olfactory sheath of patient's nose, which the fragment is isolated in the course of otolaryngologic endoscopic surgical manipulation. Preparations of hematopoietic stem cells, which are the source of EC, as well non-hematopoietic stem cells found in mononuclear fraction (MN) are produced according to the technique described in the RU patent No. 2283119 and in the registration certificate of Federal Service of Healthcare and Social Development Surveillance No. φC-2006-/151 of July 1, 2006.

The proposed method of ACBP NEPS production and the proposede method of neurosurgical operation can be implemented only under conditions of neurosurgical operation theatre of a surgical department and anesthesiological and resuscitation department of a multi-field hospital licensed for medical activities involving high technologies in neurology, neurosurgery, hematopoietic stem cells harvest and application of cell therapeutical methods by neurosurgeons and intensivists trained for cell therapy. The hospital should be equipped with present-day diagnostic means (MRI, spiral computer tomography, angiographic equipment, cytofluorimeter, blood separator) or to have agreements with institutions equipped with these means. A laboratory certifying autologous hematopoietic stem cells of peripheral blood (AHSCPB), NSC, NGEC and EC must be licensed as a laboratory authorized for cell techniques application, meet the GLP (Good Laboratory Practice) standard and have an opportunity to perform complete analysis of a cell preparation in the scope recommended by European Bone Marrow Transplant Registry. The laboratory must have an opportunity to test biopreparation for sterility, toxicity, culturing the material, i.e. be equipped with a sterile box with a laminar safety cabinet, CO₂-incubator, bifocal microscope and a kit for culturing. The personnel must be additionally trained in transfusiology.

The procedure of use of of the proposed ACBP NEPS consists of several consecutive stages:
I - specific examination of a patient;
II - harvest and preparation of biomaterial (including the proposed method of the ACBP NEPS production) and
III - reconstructive neurosurgical operation for spinal cord or brain with intraoperative preparation of the ACBP NEPS and its implantation.

### Stage I. Specific Patient Examination

At this stage, the requisite clinical and paraclinical examination of a patient according to standard protocol is performed. Diagnostic parameters of the site of lesion are analyzed, the strategy of tissue engineering for brain or spinal cord is developed, indications and contraindications for ACBP NEPS implantation are established, as well as the time schedule for every stage adjusted for the results of examination. If necessary, cell transplantation is mathematically modeled and the volume of the cell preparation to prepare the ACBP NEPS according to MRI data is planned. This stage must not be formal testing and examination. At this very stage, absolute and relative contraindications are determined, as well as medical and social prognosis of the surgery in tissue engineering in brain and spinal cord. As far as intramedullar and/or intracerebral introduction of NCS and NGEC preparations is concerned, main attention should be given to immunochemical characteristic of blood and CSF of the patient. Additional introduction of neurospecific proteins during transplantation of neurons and neuroglia-containing cells combined with initially high value of neurospecific antigens in the patient's blood and CSF can deteriorate available destructive process in CNS. Simultaneously, introduction of neurospecific proteins during transplantation of neurons and neuroglia-containing cells combined with antibodies to neurospecific antigens in the patient's blood and CSF can trigger or exacerbate autoimmune process, and in certain cases autoimmune lysis of injured tissue. The patients with antibodies to neurospecific proteins and immunological deficit are not recommended as candidates for such surgeries.

### Stage II. Harvest and Preparation of Biomaterial

### Composition of the heterogeneous implantable gel Sphero®GEL

The *Sphero*®GEL composition is produced according to the method described in the mentioned RU patent No. 2283119. It is produced in injection formulation in syringes of 1, 2 and 5 ml.

At room temperature, the heterogeneous matrix *Sphero*®GEL is elastic mass stable at long storage, not susceptible to syneresis (separating out of liquid). At the temperature of 37°C, viscosity of the biopolymer matrix sharply reduces due to weakly cross linked liquid component, while the physical condition of globular (solid) component does not change. Immunogenicity of the obtained matrix is sufficiently low.

As additional components, antibiotics, antiseptics, stimulators of regeneration and anticoagulants can be introduced into the matrix immediately before surgical intervention. The antibiotics can be penicillins (such as benzylpenicillin, cloxacillin, ampicillin), cephalosporins (such as cephaloridin, cefuroxime, cefotetan, ceftazidime), carbapenems (such as meropenem), monobactams (such as aztreonam), aminoglycosides (such as streptomycin, gentamicin, amikacin), tetracyclines (such as tetracyclin, minicyclin), macrolides (such as erythromycin, azithromycin), lincosamides (such as lincomycin), antibiotics of peptide group (such as polymyxin B, polymyxin M, ristomycin, bacitracin). As the stimulators of cell regeneration, it can be used, for example, methyluracil, leucogen, adeninosine triphosphate acid (ATP), pentoxyl, potassium orotate, inosine, etadenum.

Antibacterial and antiviral components as well as antiaggregational agents can be additionally introduced into the *Sphero*®GEL composition. The stimulators of cell regeneration are infused into the *Sphero*®GEL directly in a surgical room in the volume not exceeding 0.2 µl per 1 ml of the matrix.

The *Sphero*®GEL matrix introduced directly in the injury site is in time replaced by initial tissue without a scar formation.

Advantages of the *Sphero*®GEL matrix used for implantation in injured brain and spinal cord are as follows:
- multifunctionality (supporting and trophic functions for cell cultures and stimulation of axon regeneration and neovascularization);
- high biocompatibility of the final product as well as of the products of its biodegradation on protein and cell levels;
- capability to stimulate proliferation and differentiation of neural cells;
- regulated period of biodegradation varying from several weeks to several months (the final products of the biodegradation are water and carbon dioxide);
- cavitation capacity at immediate contact with biological media, that provides for neovascularization);
- possibility of sterilization without changing medical and biological properties.

### Processing and production of the biocomposition of cell preparations

### Production of stem cells

The procedure can be conditionally subdivided into two stages namely mobilization of stem cells into peripheral blood and stem cell harvest.

### Mobilization of stem cells into peripheral blood

To increase the number of stem cells in peripheral blood, a donor gets 8 subcutaneous injections of granulocyte colony-stimulating factor (G-CSF) every 10 to 12 hours for 4 days. G-CSF is a pharmaceutical obtained by gene engineering and absolute analogous to human factor. The first three days, the dose makes 2.5 µg/kg and the last day, the dose is doubled. The blood is tested daily and ultrasound examination of abdomen is done at day 4 to 5.

### Stem cells harvest

Stem cells are harvested at day 5 after stimulation with G-CSF in a blood separator of COBE-spectra type using a disposable system for separation and standard solutions. The procedure lasts 3 to 4 hours depending on speed of the procedure, weight of the patient and blood test results. In course of the procedure, blood is sampled from a vein, processed inside the separator, the stem cells are sampled and the rest components of blood are returned to the donor through the other vein. Veins are accessed by the puncture of two peripheral veins or dual-lumen central catheter inserted in subclavian vein for the séance. Average volume of the obtained material varies from 300 to 400 ml. The gathered material is evaluated by two parameters: according to total number of nuclear cells (NC) in the sediment and according to CD34+ cells per every kilogram of the patient's weight. NC in sediment are determined by counting in Gorjaev's chamber before any manipulations. The percentage of CD34+ in the cell preparation obtained in the course of cytapheresis is determined by flow cytometry method.

### Specification of peripheral stem cells

The subpopulation of CD34+ is established by cytofluorimetry with the method of triple-labeling (simultaneous staining of the cells with antibodies to three different antigens loaded with various dyes).

### Standardization of the preparation

The number of precursor cells is determined by cytofluorimetry in direct immunofluorescence test (DIT).

The method of double labeling is used with simultaneous staining of cell substrate with monoclonal antibodies (MCA) to CD34 antigen that is the main marker of hematopoietic stem cells pool, and to CD45 molecule that is a common leukocyte antigen characteristic for all hematopoietic stem cells. Such method permits direct calculation of the ratio of CD34+ cells and all hematopoietic (CD45+) cells in the product.

To evaluate non-specific binding, a part of the cells is stained with isotypic controls such as conventional mouse immunoglobulins IgG1 of isotype (IgG1), labeled with dyes analogous to the label of the used monoclonal antibodies (PE, FITC, PerCP).

### Preparation of cell tests

Before reaction, the cells of peripheral blood and cytapheretic product are cleared from erythrocytes by standard lysis and further washing in buffered saline with bovine serum albumin (BS-BSA) by centrifuging at 1,000 g for 5 minutes. BS-BSA can be replaced with Hank's solution or 199.

### Method of lysis of erythrocytes:

1.2 ml of the lysing solution is added to 0,2 to 0.5 ml of the cell sediment, mixed and incubated till the solution is clear (laked).
2. The cells are washed twice by 199 medium in centrifuge (1000 g, 5 to 7 min).

The selected cells are tested by DIT in 96-socket plate and to count peripheral HSC in any product, a plate with 3 sockets is used:
- Unstained cells;
- Cells stained by isotypic controls labeled according to the labels of the used MCA
- Cells stained by the MCA both to antigen CD34 and CD45.

It should be especially noted that MCA to CD34 were preferably phycoerythrin (PE) or peridin chlorophyll (PCP) labeled. Fluorochrome data have a higher level of specific signal as compared with fluorescein isothyocyanate (FITC).
To count CD34+ cells, antibodies to CD34 of HPCA-2(8G12) clone, isotype IgG1 are optimal.

Thus, the reference panel is the following:
1. IgG1 PE control+ IgG1 FITC control;
2. IgG1 PE control+MCA to CD45 FITC;
3. MCA to CD 34 PE (HPCA-2) + MCA to CD45 FITC.

### DIT testing

1. No less than 500,000 cells per a socket are inserted.
2. Further, the cocktail of antibodies according to the reference panel is introduced into the socket and carefully resuspended by a pipette. 10 µl MCA per a socket is taken, total number of MCA in the socket is 20 µl.
3. The cells are incubated with antibodies for 30 minutes at 4°C (lower shelf of a conventional fridge).
4. Incubation over, the cells are twice washed from unconjugated antibodies by 1,000 g centrifugation for 5 to 7 minutes.
5. The cells are put into special plastic tubes to count on a flow cytometer.
6. The volume of cell suspension in every tube is brought to 200 to 500 µl by adding PBS-BSA.

The count should be done immediately after the testing.

### Count and record on flow cytometer

Reaction is assessed on a flow 5-parameters cytometer. The scheme is applicable for a flow cytometer of any configuration.

CD34+ cells in peripheral blood represent a minor cell population. Even under the condition of preliminary hematopoietic stimulation, the maximal percentage of these cells is 1.0 to 3.0%. Therefore, to count these cells, no less than 20,000 cell events must be accumulated in each analized sample.

Cell collection and analysis is performed in the gate of CD45+ cells, which includes all hematopoietic cells. Here gate the means the event accumulation region restricted by certain parameters. In this case, SSC/FL-1(CD45FITC), i.e. abscissa axis of a dot cytogram will show all CD45+ events. On ordinate axis (SSC parameter - side light scattering) the cell events will be located according to their granularity (a cytometric term, not a morphologic).

The count of absolute number of CD34+ in 1 µl of blood and of cytoconcentrate was fulfilled on the baseline of leukocytes in hemogram at the day of testing.

### Principle of detecting CD34+cells in hematopoietic tissue. Recording samples

### 1. Region of analysis/gate selection

Menu of cell samples registration *Acquisition* is open on the appliance. At the first stage in *setup* mode (mode of cell sample review without recording), samples No. 1 (IgG1PE+, IgG1 FITC) and No. 2 (IgGa1 PE +CD45 FITC) are viewed and CD45+ events are detected. These events are located to the right from 10¹ values (average threshold value of specific signal level for FITC fluorescein) on x-axis - FL-1 (CD45+ cells) and are clearly visualized in comparison with control sample No. 1.

According to sample No. 1, all the region located to the right from major cell density is confined, that is gate that includes only CD45+ events in sample No. 2 and contains minimum of cell events in sample No. 1.
2. The appliance is switched to recording samples mode (*Normal*) and sample No. 1 is collected and recorded per 20,000 cell events without a gate (all cell population). Recording of the sample in the gate is impractical as MCA to CD45 antigen are absent in it. As shown above, the sample is required for correct selection of the gate containing only CD45+ events.
3. Samples Nos. 2 and 3 are collected and registered in gate CD45+. The gate is identical both for sample No. 2 and for sample No. 3, i.e. gate parameters are not changed during sampling. Minimum of cell events equals 20,000 for each sample.

### Recorded samples analysis

1. The appliance is switched to the menu of recorded samples analysis (*Analysis*)*.* Then sample No. 2 cytogram (dot-plot) is opened at SSC/FL-2 parameters, in R1 gate - CD45+ (the gate was chosen previously when recording collection and was automatically transferred to analysis mode). Thus, y-axis will display SSC parameter, i.e. granularity of events in the analyzed gate, and x-axis displays FL-2 that is the detector reflecting levels of nonspecific binding for phycoerythrin stain, which levels are detected by antibodies to mouse immunoglobulin IgG1PE.
2. Control marker is set on the received cytogram, so that almost no cell events appear in the right low quadrant. In average, the values for the upper bar of the marker will make about 130 (to the right from 10²), and for horizontal marker bar, the average values will be 60 that corresponds to SSC-low, i.e. the cells with minimum cell inclusions. Therefore, the levels of nonspecific binding will be equal or close to zero.
3. Automatically switch to sample No. 3. All indications of the appliance for sample No/2 are saved, i.e. cytogram is open at SSC/FL-2 parameters, the gate chosen earlier during CD45+ collection on cell events is saved and the meanings of the control marker set on sample No. 2 are saved too. However, on this cytogram, x-axis displays specific CD45+ events and not the levels of non-specific binding as in the second sample. CD45+ percentage is displayed by the appliance automatically.

### AHSCPB cryopreservation

### AHSCPB fraction isolation

Separated cells are concentrated by centrifuging at 2,000 revolutions per minute speed for 10 min at +18°C. Plasma is maximally removed from the container by a manual plasmaextractor, the cell volume of 40 to 60 ml is left.

### Adding Cryprotector

Purified dimethyl sulphoxide (DMSO) is used as a cryoprotector of hematopoietic stem cells. Equal volume of polyglucin with DMSO is added to the obtained cells at continuous stirring. DMSO reacts with polyglucin esothermally with moderate heat liberation. DMSO concentration in polyglucin is 10 to 12%. Thus, its final concentration in the freezing material will make 5 to 6%.

Application of polyglucin permits to reduce the DMSO amount two-fold, up to 5 to 6% final concentration because polyglucin can disaggregate cells thus improving penetration of cryophylactic into the cells, moreover, polyglucin (6% dextran) is a cryophylactic per se.

### Cell Count

The next stage necessarily involves counting of nuclear cells as well as CD34+ cells to be frozen.

### Choice of Optimal Volume of Frozen Material

To provide the best conditions for cryopreservation, correlation of plastic container volume and volume of frozen material and concentration of frozen cells are important. It is determined that the optimal concentration of the frozen cells is from 40·10⁶ to 100·10⁶ 1 cells per ml.

### Cell Freezing

Depending on the final volume of frozen material, the number of polymer tubes (15 to 20) for deep freezing should be chosen. Then the cell suspension is placed into a container for cryopreservation.

To freeze the tubes with cell suspension, they are put into a programmed freezer or a laminated plywood container (10 mm of total thick) of appropriate size with a tight lid. Then the container with the material to be frozen is put into liquid nitrogen vapor at minus 165 to minus 170°C.

The speed of cooling of the material at the temperature from 0°C to minus 40°C is 1.1 degree per minute. This cryopreservation method levels crystallization plateau that usually is clearly seen at program freezing in an electronic device, and the composition of the frozen material remains unchanged when stored.

In one or two hours after the beginning of freezing, the container can be transported to a warehouse where it will be stored in liquid nitrogen or its vapor till transplantation.

### Thawing of AHSCPB and preparation of AHSCPB to administration

The product is thawed immediately before transplantation in 37 to 40°C water bath till the moment of transition of the frozen product into liquid state. Then the mobilized hemopoietic stem cells are deposited by centrifugation at 1,500 rev/min to the bottom of a centrifugal plug, the supernatant is drained and 1 ml of 0.9% NaCl physiological solution is added. The procedure is repeated twice. The product of thawed autologous stem cells can be administered within 6 hours after its preparation. If the product was not used during this time, it must be recycled.

### Isolation of NSC and NGEC

Nasal mucosa was endoscopically isolated with informed consent of a patient priory taken. Nasal cavity was anesthetized by Sol. Halasolini 0.1 to 2.0%. Under contact anesthesia with 10% Sol. Lidocaini and local anesthesia with 1% Sol. Novocaini, a 3x4 mm size fragment of nasal septum mucosa was taken. Bleeding was suppressed by cotton plugs with solution of Halazolin and hydrogen dioxide. The plug was removed 24 hours after the surgery in the absence of any nosebleed. In case of even slight bleeding, the nasal passages are repeatedly plugged for 24 hours.

### Method of NGEC obtaining and culturing

Olfactory epithelial tissue was sampled from the upper third of superior nasal meatus of adult patients. Obtained tissue was washed in Hanks' solution that contained antibiotics (Streptomycin, Penicillin) and antimycotic (Amphotericin). The tissue sample was minced and incubated in tripsin (0.05%) and EDTA (0.02%) solution (40 min, 36.5°C). The Tissue was dissociated by multiple pipetting, centrifuged and sedimented cells were washed with Hanks' solution with 5% serum. Total number of cells in the suspension and percentage of viable cells were determined in Gorjaev's count chamber by trypan blue staining. Cells (final concentration was 100,000 cells per ml) were cultured in 12-well trays on a polylysine/laminin substratum for 10 to 15 days (5% CO₂, 36.5°C) in the following medium: DMEM/F12 (Gibco), fetal calf serum 10% (Gibco), glutamine 2 mM (Gibco), glucose 0.8%, mixture of insulin, transferrin and sodium selenite (Gibco, 1:100), HEPES buffer (10 mM), human neuregulin-1 b-1/heregulin 1-b-1 EGF domain 2 ng/ml (R&D systems). Medium was changed every 3 to 4 days. Dense monolayer being developed, the cells were removed from substratum by trypsin/EDTA solution, washed with Hank's solution and reseeded into dishes with polylysine/laminine substratum (25 cm², 10,000 to 12,000 cells per cm²). After 3 to 4 passages the cells were removed from substratum by trypsine/EDTA solution, washed with Hank's solution and the obtained suspension was either used for transplantation or frozen with cryoprotector (10% serum, 90% EDTA) and stored in liquid nitrogen at minus 70°C. For transplantation, the cryopreserved cells were thawed, their viability was detected by trypan blue staining. Control tests showed no less than 90% of cells retained viability after storage.

For cytochemical identification of olfactory epithelial glial cells, a part of suspension was cultured after every passage on a cover slip (22×22 mm) in Petri dishes for a week. Primary antibodies to acid gliofibrillar protein (GFAP, 1:6; monoclonal antibodies obtained in the Immunochemical Laboratory of Serbsky's State Research Centre of Social and Forensic Psychiatry), nestin (Chemicon International, CA, 10 µg/ml) and low affinity nerve growth factor receptor (p75, Chemicon International, CA, 10 µg/ml) were used for immunocytochemical analysis. The obtained preparations were analyzed and photographed by fluorescent microscope Leica DLMB.

### Method of isolation and culturing of NSC of olfactory sheath of patient's nose

Tissue of olfactory sheath including olfactory epithelium and lamina propria is isolated from patients with a spinal cord injury and processed according to standard culture protocol.

10 to 5mm size fragments of mucosa dissected under local anesthesia from the upper part of superior nasal meatus were accepted for research. The sampled tissue was delivered to a laboratory in cool Hank's solution without Ca²⁺ and Mg²⁺ (HBSS), containing antibiotic and antimycotic agents (1:100; Gibco). Delivery time does not exceed 2 hours. After repeated wash in the same solution, blood vessels were removed from mucosa, then the tissue was minced and incubated for 40 minutes at 36.5°C in 0.25% trypsin/EDTA solution prepared on 0.01 M phosphate buffer (PBS, pH 7.4). Activity of ferments was blocked by DMEM medium (Gibco) that contained 3% of serum, the tissue was washed three times in new Hanks' balanced salt solution (HBSS, Sigma) and dissociated by repeated pipetting in nutritive medium. Medium composition: 90% minimum Eagle medium (MEM, Sigma), fetal bovine serum (FBS, Gibco, Invitrogen), 0.8% glucose, 2mM glutamine (Gibco), B27 supplement (Sigma), HEPES 20 mM, growth factors (only for primary cultures) namely fibroblast growth factor (FGF2, 1 ng/ml, Sigma), neural growth factor (NGF 2 ng/ml, Sigma).

The obtained cell suspension was centrifuged (3 minutes at 1200 revolutions per minute), the sediment was resuspended in nutritive medium of the same composition.

The number and viability of the dissociated cells were checked in Gorjaev's chamber after 0.1% trypan blue staining. Cell suspensions with 85 to 95% of viable cells were used for further culturing.

The dissociated cells (5·10⁵ cells per ml) were cultured in 12-well trays on polylysine/laminine substratum for 14 days (36.5°C, 5% CO₂). One third of nutritive medium was partially changed 2 times a week. Primary culture, after confluent cell monolayer is formed, was removed by trypsin/EDTA solution. The cells were resuspensed in nutritive medium after wash in HBSS and centrifuging. The cell suspension (10,000 to 12,000 cells per cm²) was placed into 12-well trays or dishes (square of 25cm²). So the cultures were passed 4 times till the confluent monolayer was formed. Free floating and attached to substratum neurospheres formed in the cell monolayer were selected by Pastuer pipette and dissociated by the ferment processing method described above. The selection of the neurospheres permits to separate them from accessory glial cells, fibroblasts and stromal (foot) cells attached to the substratum. The cell suspension of the neurospheres, after washing and centrifuging, was resuspended in culture medium and cultured in 12-well trays (10,000-12,000 cells per cm²) and on cover slides (18×18 mm) in Petri dishes till confluent monolayer was formed. The obtained cultures were used for cytological and immunocytochemical tests. A part of the cells of last passages was frozen in cryopreservation medium (90% serum, 10% dimethyl sulfoxide) and stored in liquid nitrogen.

### Immunocytochemical tests

The cell monolayer was fixated in 4% solution of paraformaldehyde prepared on 0.01 M phosphate buffer (pH 7.4) for 30 minutes. After PBS washing (3×10 min), the cells were incubated for 24 hours at 4°C with primary antibodies to β-tubulin (1:300; Chemicon), nestin (1:100, Chemicon) and neural specific enolase (1:100, the antibodies are obtained in our laboratory). After PBS washing, the cells were successively processed by biotinylated antibodies with avidin-biotin complex (ABC, Vector Laboratories, Inc), diaminobenzidine solution prepared on phosphate buffer (DBA 0.5 mg/ml, hydrogen dioxide 0.03%). The preparations were dehydrated and placed into synthetic resin under cover slides (Entellan, Merk).

### Method of isolation of CD 34+ endothelial cells from the suspension of mobilized stem cells

To obtain the suspension of endothelial cells (EC), standard procedure to separate stem hematopoietic cells CD34+ CD133+ from leukoconcentrate of mobilized peripheral blood and magnetic beads separation (CD34+) with further short-term standard culturing was applied. Culture medium consists of Iscove modified Dulbecco's medium with added 0.5 g/l of serum albumin, 0.39 µg/ml of human insulin and 60 µg/ml of transferrin. 100 ng/ml of stem cell factor (SCF), 20 ng/ml of Flt3 (Flt3L) and 20 ng/ml of thrombopoetin (TPO) are added to the culture medium. Culturing regimen is the following: the cells are cultured at 37°C in the atmosphere of 5% CO₂ and fresh medium and cytokines are added every three days. Period of culturing makes 5 to 7 days.

To avoid contamination, the cell preparation is purified from autologous mononuclears, erythrocytes, thrombocyttes and other blood elements. The preparation of purified mononuclears is prepared from the cryopreserved and defrozen cell preparation of mobilized autologous stem cells (MASC) by washing of 10% DMSO solution by means of triple centrifuging at 2,000 rev/min with physiological solution of 0.9% NaCl.

### Stage III. Reconstructive neurosurgical operation for spinal cord and brain, intrasurgical preparation of ACBP NEPS, implantation of ACBP NEPS

### Intrasurgical preparation of ACBP NEPS

The cell biocomposition is prepared in sterile conditions either directly in a surgical room (ex tempore) or in advance in a culture laboratory (to be implanted within 6 hours). The amount of the ACBP NEPS is defined depending on the volume, type and location of the injury. Provisional calculation of necessary amount of the ACBP NEPS is evaluated according to MRI or CT imaging results. The ACBP NEPS is prepared as follows. The cell preparations in the above-mentioned proportions are mixed in a sterile tube in required volumes and mixed with 2 to 3 syringe intakes and discharges back into the tube. The *Sphero*®GEL preparation is taken out of a refrigerator and warmed to 37.5 to 38°C. A disposable syringe of the volume corresponding to the required calculated volume of the ACBP NEPS is used. The hub is removed from the syringe and the needle cannula is closed with a rubber or plastic plug (the syringe is used as a tube). 1 ml of the *Sphero*®GEL is placed on the bottom of the syringe tube and up to 1 ml of the cell biocomposition is added. The tube is centrifuged for 2 minutes in a minicentrifuge at 2,000 rev/min. Liquid component of the biocomposition with the cells is infused into the *Sphero*®GEL during centrifuging. If a larger volume of the ACBP NEPS is to be prepared, the above mentioned procedure is performed in layers in the syringe tube by placing the *Sphero*®GEL and the cell biocomposition in layers as in a sandwich and then centrifuged for 2 to 3 minutes. The hub is returned into the syringe and the air is removed therefrom. A plastic sterile venous catheter is put on the syringe cannula, which catheter will be used for implantation. Within 30 to 40 minutes after preparation of the ACBP NEPS, it is left in the syringe under sterile conditions at room temperature (20 to 22°C) to cool down and to acquire toothpaste consistence. Then the ACBP NEPS can be implanted.

### Reconstructive surgery for brain and spinal cord and ACBP NEPS implantation

Spinal cord defects are reconstructed with the ACBP NEPS in standard neurosurgical operation of decompressive laminectomy on the level of injury by opening of dura mater (DM) and radiculomyelolysis of the site of injury. The ACBP NEPS is placed on the surface of spinal cord defect and modeled along the injury. In case of intramedullar or intracerebral implantation, the ACBP NEPS is isolated from aggressive cerebrospinal fluid. Hence, an intramedullar cyst (cysts) is opened, drained and the ACBP NEPS is implanted inside the cyst. In case of complete anatomical dissections of spinal cord, a conduit is microsurgically modeled in the gap area of spinal cord, that is a tube-like conductor (TLC) the walls of which are formed either from DM of the patient or a hip muscle fascia of the patient or artificial arterial (aorta) graft attached to DM of the patient. The ACBP NEPS is implanted inside the TLC. Proximal and distal ends of injured spinal cord are attached (tunneled and sutured to DM) to the formed conduit.

Spinal cord plasty being completed, DM is closed and sutured with internal part of autologous DM or artificial DM, then the site of DM plasty is covered with standard biopolymer glue (like Tissucol®). Implantation of the ACBP NEPS into brain defects is done after evacuation of the content (CSF, detritus etc) out of cystic cavities of brain during open neurosurgical operations, as well as in course of functional neurosurgical interventions (stereotaxis, endoscopic manipulations etc.) and implantation of the ACBP NEPS in vegetative ganglia. It was shown that the ACBP NEPS degrades within 12 to 24 months, and the site of prosthesing is replaced by fibers of autologous nervous tissue partially or completely. MRI imaging of the site of operation 8 to 12 months post the intervention allows for verification and evaluation of the morphological tissue structures of brain and spinal cord.

After the ACBP NEPS implantation, the patient is under observation for 10 days 24 hours a day. An intensivist together with an attending doctor should control the condition of the patient to assess complication risks. Besides, regular observation of a neurosurgeon and a neurologist is recommended, who should work in close cooperation with hematologists, transplantologists, immunologists and laboratory specialists.

The main efficacy criteria of this intervention are improvement of neurological symptoms (motor, sensation and bowel and bladder functions). The period of first manifestation of expected results is individual and depends on the volume of brain/spinal cord injury, period post injury, level of compensation of injured functions. Therapuetical efficiency varies from 7 days to 48 months after tissue engineering surgery and is evaluated by the indexes of ASIA (American Spinal Injury Association), FIM (Functional Independence Measurement) and neurophysiological methods of testing (cerebral EEG mapping, transcranial magnetic stimulation, somatosensory evoked potentials, electroneuromyography and complex urodynamic testing).

### Industrial applicability

Efficiency of the present invention was evaluated in the patients with consequences of brain and spinal cord injuries in comparison with conventional surgical therapy. To objectify received clinical results, specific ASIA and FIM indexes were involved, as well as data received by MRI, electroneuromyography, cerebral EEG mapping, complex urodynamic testing, immunochemical tests of blood and CSF. The trial was done in 50 patients. Surgeries in tissue engineering with the ACBP NEPS implantation were given to 30 patients with severe traumatic disease of spinal cord. All the patients enrolled into the trial were subdivided into two groups. The 1^{st} group (main, 30 patients) consisted of the patients who had been operated with the ACBP NEPS implantation, the 2^{nd} group (control, 20 patients) included patients that received conventional surgical therapy (decompressive laminectomy, microsurgical radiculomyelolysis, drainage of intramedullar cysts, DM plasty). Distribution of the patients by age and gender is shown in Table 1.

**Table 1**

| **Distribution of patients by age and gender** | | | | | | | | |
|---|---|---|---|---|---|---|---|---|
| Age (years) | 1^{st} group | | | | 2^{nd} group (control) | | | |
| | Male | | Female | | Male | | Female | |
| | Number | % | Number | % | Number | % | Number | % |
| 14-19 | 2 | 9 | 1 | 12.5 | 3 | 20 | - | - |
| 20-30 | 11 | 50 | 2 | 25 | 5 | 33.3 | 5 | 100 |
| 31-40 | 7 | 31.8 | 4 | 50 | 6 | 40 | - | - |
| 41-50 | 1 | 4.5 | 1 | 12.5 | 1 | 6.7 | - | - |
| 51-60 | 1 | 4.5 | - | - | - | - | - | - |
| Total | 22 | 100 | 8 | 100 | 15 | 100 | 5 | 100 |

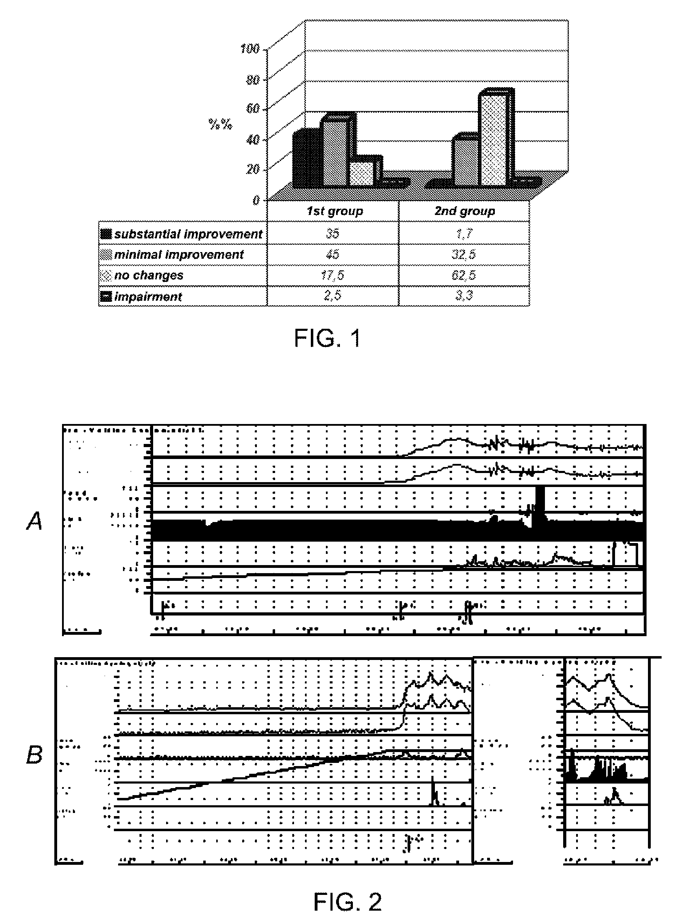

Comparison of efficiency of SC tissue engineering and the ACBP NEPS implantation for traumatic disease of SC and brain with conventional surgical therapies is demonstrated in Fig.1. The 1^{st} group shown here presents the patients that received surgical therapy with the ACBP NEPS implantation according to the present invention; the 2^{nd} group is the patients of the control group that received conventional surgical interventions. Efficiency of SC tissue engineering with the ACBP NEPS implantation for SCI was statistically significant (p<0.05) as compared to the control group. Significance (p<0.05) was calculated by x² method.

Fig. 2 shows changes of urodynamic values in patient V before and after the ACBP NEPS implantation according to the present invention (A - urogram before the therapy, B - urogram after the ACBP NEPS implantation).

The received data are shown in Tables 2 and 3 and accompanied by clinical Examples 1 and 2.

**Table 2**

| Results of therapy of SCI patients after tissue engineering surgeries with ACBP NEPS implantation. | | | | | | | | | | | | |
|---|---|---|---|---|---|---|---|---|---|---|---|---|
| No | Patient | Injury level | Time post injury (years) | ASIA, FIM indexes before therapy | | ACBP NEPS composition* | ASIA, FIM indexes post therapy | | Time post treatment (months) | Sensation | Motor restoration | Bowel and bladder control |
| 1 | B-ev N.V. | C5 | 4 | A | 46 | No. 2 | B | 48 | 11 | T1 | Complete | Partial |
| 2 | P-us M. | T7 | 2 | A | 46 | No. 4 | A | 46 | 10 | T10 | Partial | Partial |
| 3 | Sch-ba F. | C7 | 8 | A | 37 | No. 3 | A | 37 | 11 | T3 | Partial | Partial |
| 4 | T-syan A. | T1 | 2 | A | 24 | No. 3 | A | 24 | 11 | T5 | Partial | Partial |
| 5 | R-ska D. | T11 | 2 | A | 31 | No. 4 | C | 37 | 10 | Complete | Partial | Complete |
| 6 | K-kin A.V. | T10 | 3 | A | 46 | No. 2 | B | 46 | 10 | L2 | Partial | Complete |
| 7 | Al Azzi D-11 | C5 | 3 | A | 57 | No. 4 | A | 57 | 8 | C7 | Partial | Complete |
| 8 | N-shin A.A. | C3 | 7 | A | 30 | No. 2 | A | 32 | 8 | C7 | Partial | Complete |
| 9 | M-t A. | T10 | 2 | A | 18 | No. 4 | A | 18 | 6 | T12 | No | No |
| 10 | D-es P. | C5 | 3 | A | 49 | No. 4 | A | 49 | 1 | T6 | Partial | No |
| 11 | A-yan E. | T5 | 5 | A | 24 | No. 4 | A | 16 | 17 | T5 | Partial | Complete |
| 12 | B-chuk N.N. | T12 | 6 | A | 25 | No. 4 | A | 21 | 14 | Complete | Partial | Partial |
| 13 | Sh-lekh N. | T7 | 5 | A | 35 | No. 4 | B | 27 | 30 | T10 | Partial | Partial |
| 14 | H-m N. | T5 | 2 | A | 24 | No. 4 | A | 18 | 12 | T12 | Partial | Complete |
| 15 5 | D-ov T. | T4 | 5 | A | 22 | No. 3 | B | 17 | 31 | T7 | No | Complete |
| 16 | Ya-in S.A. | C7 | 4 | A | 53 | No. 3 | A | 50 | 30 | T3 | No | Complete |
| 17 | Sh-ga Ya. | T4 | 16 | A | 36 | No. 4 | C | 24 | 27 | Complete | Partial | Complete |
| 18 | Kh-osh P.M. | T5 | 4 | A | 44 | No. 3 | A | 38 | 24 | T7 | Partial | Partial |
| 19 | L-kov A.S. | T5 | 6 | A | 43 | No. 3 | A | 39 | 26 | T8 | Partial | Partial |
| 20 | A-ev Ye.L. | T6 | 2 | A | 31 | No. 3 | A | 27 | 26 | T10 | Partial | Complete |
| 21 | S-ds E. | C7 | 5 | A | 46 | No. 2 | B | 44 | 15 | C7 | No | Partial |
| 22 | V-ev A.V. | T2 | 2 | A | 44 | No. 2 | B | 37 | 25 | T2 | Partial | Partial |
| 23 | V-der L. | C5 | 2 | A | 66 | No. 3 | A | 66 | 25 | C7 | Partial | Partial |
| 24 | I-va O.A. | T3 | 5 | B | 54 | No. 4 | C | 39 | 25 | T6 | Complete | Complete |
| 25 | Sch-kov S.G. | T8 | 11 | B | 35 | No. 1 | C | 34 | 45 | T9 | Partial | Partial |
| 26 | O-kov A.N. | T7 | 10 | A | 28 | No. 3 | B | 27 | 44 | T12 | Partial | Complete |
| 27 | L-shin P.A. | C6 | 1 | A | 62 | No. 3 | C | 52 | 42 | T4 | Partial | Partial |
| 28 | L-va O.D. | T8 | 6 | A | 27 | No. 3 | B | 15 | 42 | T10 | Partial | Complete |
| 29 | Yu-va O.V. | T12 | 1 | A | 24 | No. 2 | B | 14 | 41 | L2 | Partial | Complete |
| 30 | V-na N.V. | L2 | 4 | A | 19 | No.1 | A | 19 | 41 | L2 | No | No |

| | | | | | | | | | | | | |
|---|---|---|---|---|---|---|---|---|---|---|---|---|
| * ACBP NEPS composition: No. 1: *Sphero*®GEL; No. 2: *Sphero*®GEL, MN and EC; No. 3: *Sphero*®GEL, NGEC and NSC; No. 4: *Sphero*®GEL, NSC, NGEC, MN and EC | | | | | | | | | | | | |

**Table 3**

| **Changes in neurological symptoms in some SCI patients after tissue engineering with ACBP NEPS implantation.** | | |
|---|---|---|
| Patient | Time post surgery (months) | Neurological progress |
| V-na | 36 | No neurological progress. After removal of metal construction, the spine became unstable. |
| O-kov | 24 | Deep and touch sensation improved. Movement appeared in left toes. Full control of bladder, bowel and sexual function restored. |
| Sch-kov | 32 | No significant clinical effect was observed. |
| Yu-va | 19 | Able to stand, to "fix" knees, to walk 12 steps. Bowel and bladder control restored, deep and touch sensation improved. |
| L-va | 15 | Level of sensation moved 25 cm down. Able to move legs when asked, control bladder and bowel. Able to make 15 backwards and 2 to 3 steps forward. |
| D-ov | 6 | Deep sensation restored, as well as function of bowel and bladder. Able to cycle on a stationary bike without assistance. |
| Ya-kin | 5 | Weakness in right arm increased, then restored in 7 months, mosaic improvement of sensation |
| V-ev | 4 | Right arm movement improved, sensation in back and buttocks area restored, as well as sexual function. |
| L-kov | 5 | Controls bowels and anus, restored thermal regulation and sweating in lower extremities. Restored movement in toes. |
| A-ev | 7 | Controls movement of both legs, the elements of deep sensation appeared. |
| Sh-ga | 8 | Controlled movement in right leg appeared, no pains in the site of injury. Thermal regulation and sweating fully restored, as well as bowel and bladder function. Able to cycle on a stationary bike without assistance. |
| I-va | 6 | Knee (S<D) and Achilles(S<D) reflexes developed, leg spasticity increased, bowel and bladder functions fully restored. |
| L-shin | 20 | Able to turn in his/her bed wothout assistance, to sit. Epicystostoma is removed. Arm movement has improved by 50%, bowel and bladder functions restored. Able to eat, drink and serve himself without assistance. |

Hence, methods of tissue engineering with the ACBP NEPS implantation according to present invention for severe traumatic injuries of brain and spinal cord (including completely severed spinal cord) can be considered as the method of choice, the efficiency of which reaches 45% in the cases when all traditional methods and treatment approaches are practically inefficient. Conventional surgical approach to the therapy of severe chronic brain and spinal cord injuries does not result in significant improvement of neurological symptoms, while application of the proposed medical technique considerably improves life quality of the patients with severe SCI and brain injury and significantly ameliorates functional and social independence of the patients (see Tables 2, 3)

To date, the proposed method of neurosurgical operation is the only efficient therapy of patients with severe chronic injuries of spinal cord and brain, including complete anatomical dissection of spinal cord (with the gap up to 5 cm) and can lead to significant improvement of their condition, life quality as well as better social adaptation. Possible return of the patients after brain and/or spinal cord injury to normal social activity results in higher economical efficiency considering both return to work activity and reduction of expenses for long-term and inefficient treatment.

### Clinical Example No. 1

Patient Sch-ga, age 50. Complains: steady girdle pains on the level of left costal arch, absence of voluntary movements in the legs, absence of all types of sensation from the level of inguinal fold, constipation and no controlled urination (catheterizes herself for 14 years).

The patient received the injury on thoracic level of spinal cord in road accident in 1991: complicated compression fracture of ThIII and ThIV vertebrae. In early period lower paraplegia was observed, lower paraanesthesia from Th15 segment. No surgery was given to thoracic level of spine. The patient has repeatedly received specialized training in rehabilitation centers with no notable effect.

The patient was admitted to Neurovita Clinic October 02, 2006 to be enrolled into the research program "New Cell Technologies to the Medicine". At the admission time the condition of the patient was compensated. Skin and visible mucosa were clean, of normal color, wet. Regional lymph nodes were not palpated. Chest was regular shaped. Breath was independent, adequate, and free. Respiratory rate was 16 per minute. Pulse was rhythmical, of a satisfactory quality, 88 per minute. Blood pressure was 120/60. Heart tones were clear, rhythmical. The tongue was pink, wet. Abdomen was soft, painless. The liver was not enlarged, spleen was not palpated. The kidneys were not palpated in the seated position. Sense organs and endocrine glands demonstrated no rough defects. Urination was performed with the help of Foley catheter. Defecation was regular every other day with laxative suppositories. The consciousness was clear. The patient was time, space and personality oriented. Pupils were round, D=S, photoreaction was intact, symmetric. Ocular motility was normal, no nistagmus. No face sensation disorders. The face was symmetric, soft palate was symmetric, moving. Swallowing and phonation was intact. No atrophies, pareses, myofacsiculations of trapezius and nodding muscles. The tongue was on the median line, no atrophies, no fasciculations. Coordination tests were performed satisfactorily. Muscle tone in proximal and distal muscle groups was not changed. No hand muscle hypotrophy. Tendon and periosteal reflexes were moderate, no significant difference between the sides. Arm muscle force scored 5. Abdominal reflexes are not evoked. Muscle tone of leg proximal and distal muscle groups was elevated according to spastic type, 3 points by Ashworth index. Notable hypotrophy of calf muscle was observed. Tendon and periosteal reflexes wewre brisk, D=S. Pathological reflexes of feet, feet and kneecap clonuses were observed. Movements of hip flexor muscles were intact (1 point), as well as of knee extensor muscle (2 points). Pain and temperature para-anetshesia from the level of Th12, tactile paraanesthesia from Th4 level. Bowel and bladder dysfunction manifested in constipations and urine incontinence. No meningeal syndrome. Functional evaluation by ASIA index: 56*42*46, level of spinal cord injury - A (complete). Functional evaluation by FIM index was 36%.

The results of complex examination of the patient permitted her inclusion into research program "New Cell Technologies to Medicine". The patient received the course of MASC transfusions into subarachnoid space according to the program. The received therapy led to positive effect manifested in the sensation of bladder filling.

The patient received surgical intervention on September 26, 2005 in NeuroVita Clinic: ThII-ThIII-ThIV laminectomy, meningoradiculomyelolysis, tissue engineering of spinal cord with the application of the collagen containing heterogeneous matrix *Sphero*®GEL with implanted autologous ensheathing gliaolfactory cells (2.8·10⁶), bypassing of spinal canal with frame-mounted vascular prosthesis «Gore Tex». Postsurgical period was normal, the wound healed with primary adhesion.

Control examination in two years post surgery demonstrated compensated condition of the patient. Skin and visible mucosa were clean, of normal color, wet. Regional lymph nodes were not palpated. Breath was independent, adequate, and free. Respiratory rate was 14 per minute. Pulse was rhythmical, of a satisfactory quality, 86 per minute. Blood pressure was 110/70. Heart tones were clear, rhythmical. The tongue was pink, wet. Abdomen was soft, painless. The liver was not enlarged, spleen was not palpated. The kidneys were not palpated in the seated position. Sense organs and endocrine glands demonstrate no rough defects. Urination was performed with intermittent catheterization. Defecation was regular every other day with laxative suppositories. The consciousness was clear, time, space and personality oriented. Pupils were round, D=S, photoreaction was intact, symmetric. Ocular motility was normal, no nistagmus. No face sensation disorders. The face was symmetric, soft palate was symmetric, moving. Swallowing and phonation was intact. No atrophies, pareses, myofacsiculations of trapezius and nodding muscles. The tongue was on the median line, no atrophies, no fasciculations. Coordination tests were performed satisfactorily. Muscle tone in proximal and distal muscle groups was not changed. No hand muscle hypotrophy. Tendon and periosteal reflexes were moderate, no significant difference between the sides. Arm muscle force scores 5. Abdominal reflexes were low, D=S. Muscle tone of leg proximal and distal muscle groups was elevated according to spastic type, 2 points by Ashworth index. Moderate hypotrophy of calf muscles was observed. Tendon and periosteal reflexes were moderate, D=S. No pathological reflexes no clonuses were observed. Hip flexor muscles scored 3 points, knee extensors - 2 points, dorsal feet flexors score 2 points, extensors of the 1^{st} toe - 2, toe flexors - 2 points. The patient can "fix" the knees during verticalization. Pain and temperature para-anetshesia from the level of L3, tactile para-anesthesia from L3 level. Sensation of bladder feeling developed, as well as voluntary control under urination. The patient does not use catheterization or pampers. No meningeal syndrome. Functional evaluation by ASIA index: 74*98*76, level of spinal cord injury - C (incomplete). Functional evaluation by FIM index was 27%. The results of neurophysiological test showed low-amplitude, unclearly structured cerebral somatosensory evoked potentials during stimulation of both lower extremities. The amplitude of M-response from lower extremities muscles increased.

### Clinical Example No.2

Patient R-ska, age 37. Complains: absence of voluntary movements in the legs, absence of all types of sensation from the level of inguinal fold, constipation and no controlled urination.

The patient received the injury on thoracic level of spinal cord in road accident in 2005: complicated compression fracture of ThXI and ThXII vertebrae. In early period, lower paraplegia was observed, lower paraanesthesia from Th11 segment. Received 3 surgical interventions: 1. Decompressive laminectomy, transpedicular stabilization on the level of ThX-LII (November 2005, Krakow, Poland); 2. Interbody fusion from anterio-lateral acces suing mesh and fixing plate on the level of ThXI-ThII (January, 2006, Tarnow, Poland); 3. Dismantling of transpedicular system and installation of rigid transpedicular stabilization on the level of ThX-LI-LII (February, 2006, Tarnow, Poland). The patient repeatedly received rehabilitative treatment in reconditioning centers of Poland and Germany with no significant effect.

The patient was admitted to Neurovita Clinic October 02, 2006 to be enrolled into the research program "New Cell Technologies to Medicine". At the admission time, the condition of the patient was compensated. Skin and visible mucosa were clean, of normal color, wet. Regional lymph nodes were not palpated. Chest regularly shaped. Breath was independent, adequate, and free. Respiratory rate was 18 per minute. Pulse is rhythmical, of a satisfactory quality, 82 per minute. Blood pressure was 110/70. Heart tones were clear, rhythmical. The tongue was pink, wet. Abdomen was soft, painless. The liver was not enlarged, spleen was not palpable. The kidneys were not palpable in the seated position. Sense organs and endocrine glands demonstrated no rough defects. Urination was performed with the help of Foley catheter. Defecation was regular every 2 to 3 day with laxative suppositories. The consciousness was clear. The patient was time, space and personality oriented. Pupils were round, D=S, photoreaction was intact, symmetric. Ocular motility was normal, no nistagmus. No face sensation disorders. The face was symmetric, soft palate was symmetric, moving. Swallowing and phonation was intact. No atrophies, pareses, myofacsiculations of trapezius and nodding muscles. The tongue was on the median line, no atrophies, no fasciculations. Coordination tests were performed satisfactorily. Muscle tone in proximal and distal muscle groups was not changed. No hand muscle hypotrophy. Tendon and periosteal reflexes were moderate, no significant difference between the sides. Arm muscle force scores 5. Abdomen reflexes were absent. Muscle tone of leg proximal and distal muscle groups was elevated according to spastic type, 3 points by Ashworth index. Notable hypotrophy of calf muscle was observed. Tendon and periosteal reflexes were brisk, with expanded reflexogenic zones, D=S. Pathological reflexes of feet, feet and kneecap clonuses were observed. Lower spastic paraplegia. Pain and temperature para-anetshesia from the level of L1, tactile paraanesthesia from L1 level. Bowel and bladder dysfunction manifested in constipations and urine retention. No meningeal syndrome. Functional evaluation by ASIA index: 50*74*74, level of spinal cord injury - A (complete). Functional evaluation by FIM index was 31 %.

The results of complex examination of the patient permitted her inclusion into research program "New Cell Technologies to Medicine". The patient received the course of transfusions of MASC into subarachnoid space according to the program. Received therapy led to positive effect manifested in the sensation of bladder filling and urge for urination.

The patient received surgical intervention March 07, 2007 in NeuroVita Clinic: dismantling of transpedicular stabilizing system in ThX-LI-LII levels. LI laminectomy, meningoradiculomyelolysis, tissue engineering of spinal cord with the application of the collagen containing heterogeneous matrix *Sphero*®GEL with implanted autologous ensheathing gliaolfactory cells (3.5·10⁶) and mobilized autologous stem cells (EC and MN). Arachnoid membrane modeling. Dura mater plasty. Stabilizing system on the level of ThX-LI-LII restored. Postsurgical period was normal, the wound healed with primary adhesion.

The patient received individual rehabilitation.

Control examination in a year post surgery demonstrated compensated condition of the patient. Skin and visible mucosa are of normal color, wet. Regional lymph nodes are not palpable. Chest is regularly shaped. Breath is independent, adequate, and free. Respiratory rate was 16 per minute. Pulse was rhythmical, of a satisfactory quality, 86 per minute. Blood pressure was 120/80. Heart tones were clear, rhythmical. The tongue was pink, wet. Abdomen was soft, painless. The liver was not enlarged, spleen is not palpable. The kidneys were not palpable in the seated position. Sense organs and endocrine glands demonstrate no rough defects. Urination was performed with intermittent catheterization. Defecation was regular, every other day with laxative suppositories. The consciousness was clear, the patient was time, space and personality oriented. Pupils were round, D=S, photoreaction was intact, symmetric. Ocular motility was normal, no nistagmus. No face sensation disorders. The face was symmetric, soft palate was symmetric, moving. Swallowing and phonation was intact. No atrophies, pareses, myofacsiculations of trapezius and nodding muscles. The tongue was on the median line, no atrophies, no fasciculations. Coordination tests were performed satisfactorily. Muscle tone in proximal and distal muscle groups was not changed. No hand muscle hypotrophy. Tendon and periosteal reflexes were moderate, no significant difference between the sides. Arm muscle force scores 5. Abdominal reflexes are absent. Muscle tone of leg proximal and distal muscle groups was elevated according to spastic type, 1 point by Ashworth index. Moderate hypotrophy of calf muscles was observed. Tendon and periosteal reflexes were moderate, D=S. No pathological reflexes no clonuses were observed. Movements in hip flexor muscles developed and scored 3 points, knee extensors scored 3 points, dorsal feet flexors scored 3 points, extensors of the 1^{st} toe - 3 points, toe flexors - 2 points. The patient could independently raise right leg to the height of 50 to 60 cm and make a semi-circle around limb axe. Pain and temperature para-anetshesia from the level of L2, tactile parahypestesia from L2 level. Sensation of a full bladder developed No meningeal syndrome. Functional evaluation by ASIA index: 78*94*78, level of spinal cord injury - C (incomplete). Functional evaluation by FIM index was 27%. The results of neurophysiological test showed increase of the amplitude of activity potential of sensory fibers in lower extremities, increase of excitation speed along sensor fibers on both sides, increase of H-reflex habituation after stimulation on both sides.

## Claims

1. An implantable neuroendoprosthetic system to replace defects of a brain, spinal cord and vegetative nervous system in a mammal in reconstructive neurosurgical operations, wherein the system is an elastic cell-biopolymer biologically active mass produced from a heterogeneous collagen-containing matrix for implantation and a biocomposition of cell preparations of various types of autologous cells of a patient.

2. The implantable system of Claim 1 wherein said biocomposition comprises, in a NaCl solution, neural stem cells (NSC), neuroglial ensheathing cells (NGEC), endothelial cells with CD34+ marker (EC) and purified mononuclears (MN) in the following ratios (in parts according to numbers of the cells): 0.8 to 1.2 of NSC; 1.6 to 2.4 of NGEC; 4 to 6 of EC; 4000 to 6000 of MN.

3. The implantable system of Claim 2 wherein 0.5 to 1.3% solution of NaCl is used.

4. The implantable system of claim 1 wherein a composition of a heterogeneous implantable gel *Sphero*®GEL is used as said heterogeneous collagen-containing matrix.

5. The implantable system of any of Claims 1 to 4 wherein numbers of said cells contained in 0.5 to 1 ml of 0.9% NaCl solution per 1 ml of said heterogeneous collagen-containing matrix are as follows: 10⁶ of NSC; 2·10⁶ of NGEC; 5·10⁶ of EC; 5·10⁹ of MN.

6. The implantable system of Claim 1 wherein said biocomposition further comprises stimulators of cell regeneration, nerve growth factors and vascular growth factors.

7. A method of production of an implantable neuroendoprosthetic system to replace defects of a brain, spinal cord and vegetative nervous system in a mammal in reconstructive neurosurgical operations, comprising perfusion of a biocomposition of cell preparations of various types of autologous cells of a patient into a heterogeneous collagen-containing matrix for implantation to obtain an elastic cell-biopolymer biologically active mass.

8. The method of Claim 7 wherein said perfusion is carried out by centrifugation.

9. The method of Claim 8 wherein said centrifugation is carried out within 1.5 to 2.5 minutes at 1,500 to 2,500 revolutions per minute.

10. The method of Claim 7 wherein said biocomposition is prepared from cryopreserved cell preparations that, immediately before production of said implantable neuroendoprosthetic system, are thawed by a water bath at 37 to 40°C and then washed at least twice in a physiological NaCl solution.

11. The method of any of Claims 7 to 10 wherein said biocomposition comprises, in a NaCl solution, neural stem cells (NSC), neuroglial ensheathing cells (NGEC), endothelial cells with CD34+ marker (EC) and purified mononuclears (MN).

12. The method of claim 11 wherein a source of said NSC and NGEC is olfactory sheath of a nose of a patient, and a source of said EC and MN is either a bone marrow of the patient or a leukoconcentrate of mobilized autologous stem cells of the patient, wherein the leukoconcentrate is obtained during separation of the patient's peripheral blood after patient stimulation with a granulocyte colony-stimulating factor.

13. The method of claim 1 wherein stimulators of tissue regeneration, nerve growth factors and vascular growth factors are added in said biocomposition.

14. The method of Claim 1 wherein it is carried out in sterile conditions either directly in an operation room (ex tempore), or in a culture laboratory.

15. A method of a reconstructive neurosurgical operation to replace defects of a brain, spinal cord and/or vegetative nervous system in a mammal, comprising implantation of a neuroprosthetic system in the form of an elastic cell-biopolymer biologically active mass into a defect of neural tissue, wherein said mass is obtained from a heterogeneous collagen-containing matrix for implantation and a biocomposition of various cell preparations of various types of autologous cells of a patient.

16. The method of Claim 15 wherein said biocomposition comprises, in a NaCl solution, neural stem cells (NSC), neuroglial ensheathing cells (NGEC), endothelial cells with CD34+ marker (EC) and purified mononuclears (MN).

17. The method of Claim 15 wherein a composition of a heterogeneous implantable gel *Sphero*®GEL is used as said heterogeneous collagen-containing matrix.

18. The method of Claim 15 wherein said neuroendoprosthetic system is implanted by its placing into said defect and by filling a whole volume of a cyst or a lesion of the brain and/or spinal cord with the system.

19. The method of Claim 18 wherein, after said neuroendoprosthetic system has been placed into the defect, the system is covered with an autologous muscle fascia or an artificial dura mater and/or a biodegradable synthetic polymer coat to reduce a contact of the neuroendoprosthetic system with cerebrospinal fluid of the patient.

20. The method of Claim 19 wherein an implantable biopolymer membrane ElastoPOB® is used as said biodegradable synthetic polymer coat.

21. The method of Claim 15 wherein, in case of a complete anatomical dissection of the spinal cord, said neurondoprosthetic system is implanted in the following way: a conduit is formed from an artifical arterial graft and said neurondoprosthetic system by which, at least partially, said graft is filled, said conduit is then placed in a gap between ends of the injured spinal cord and then pia mater of distal and proximal ends of the injured spinal cord is sutured to the conduit walls.

22. The method of Claim 21 wherein the length of said artificial arterial graft is equal to the length of said gap, and the width of the graft is equal to the diameter of the spinal cord in the lesion site.

23. The method of any of Claims 15 to 22 wherein said neuroendoprosthetic system is isolated from a direct aggressive influence of cerebrospinal fluid in an intramedullar or intracerebral implantation.
